# EUROPÄISCHE PATENTANMELDUNG

(11) **EP 0 711 557 A1**
(43) Veröffentlichungstag der Anmeldung: **15.05.1996**
(21) Anmeldenummer: 95810687.4
(22) Anmeldetag: 01.11.1995
(51) Int. Cl.: A61K 31/55, A61K 9/107

(54) **Pharmazeutische Formulierungsgrundlage**

(30) Priorität: 09.11.1994 CH 3375/94; 02.03.1995 CH 595/95
(71) Anmelder: CIBA-GEIGY AG, CH-4002 Basel (CH); VESIFACT AG, CH-6340 Baar (CH)
(72) Erfinder: Weder, Hans Georg, Prof. Dr., CH-8803 Rüschlikon (CH); Isele, Ute, Dr., D-79241 Ihringen (DE)

(57) **Zusammenfassung**

Die vorliegende Erfindung betrifft eine Formulierungsgrundlage für die Herstellung einer pharmazeutischen Zusammensetzung für die intravenöse Applikation eines schwerlöslichen Staurosporin-Derivats, insbesondere N-Benzoylstaurosporin. Die Formulierungsgrundlage enthält eine Kombination aus Phospholipiden, Triglyceriden und Partialfettsäureester des Polyoxyethylensorbitans als Solubilisatoren.

## Beschreibung

Die vorliegende Erfindung betrifft eine Formulierungsgrundlage für die Herstellung einer pharmazeutischen Zusammensetzung für die intravenöse Applikation eines schwerlöslichen Staurosporin-Derivats, Verfahren zur Herstellung dieser Formulierungsgrundlage und ihre Weiterverarbeitung zu einem pharmazeutischen Präparat.

Das Ausgangsmaterial zahlreicher Derivate, Staurosporin, wurde im Jahre 1977 aus den Kulturen von Streptomyces staurosporeus AWAYA, TAKAHASHI, OMURA SP. NOV. AM 2282, vgl. S.Omura et al., J. Ant. 30, 275-281 (1977), isoliert. Für das Grundgerüst wurde zuerst die relative Konfiguration und danach die absolute Konfiguration ermittelt, siehe N.Fumato et al., Tetrahedron Letters 35:8, 1251-1254 (1994). Dem besonders bevorzugten N-Benzoyl-staurosporin-Derivat, welches in der U.S.Patentschrift Nr. 5 093 330 beschrieben ist, lässt sich folgende Strukturformel zuordnen:

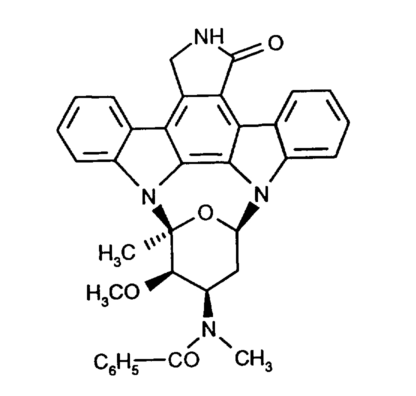

Staurosporin sowie seine Derivate, wie N-Benzoyl-Staurosporin, weisen eine starke Hemmung von Proteinkinase C auf, sie hemmen aber auch ebenso andere Proteinkinasen. Sie eignen sich therapeutisch für verschiedene Indikationen, insbesondere als Tumorhemmer, als Antiinflammatorika, als Antibiotika, bei der Behandlung von Arteriosklerose, und diversen Erkrankungen des kardiovaskulären Systems und des zentralen Nervensystems. Für Staurosporin und die meisten Derivate ist die geringe Wasserlöslichkeit charakteristisch, was ihre Verwendung für intravenöse Darreichungsformen bisher ausserordentlich erschwert.

Obwohl perorale Darreichungsformen, wie Tabletten oder Kapseln, zunehmende Bedeutung erlangen, bleiben intravenöse Darreichungsformen trotz gewisser Nachteile weiterhin relevant. Den Nachteilen, z.B. Vomahme der Verabreichung nur durch den Arzt bzw. besonders beauftragtem Hilfspersonal und Geschicklichkeitsanforderungen an die verabreichende Person, "psychologischen" Problemen beim Patienten und seinem Schmerzempfinden und hohem technischen Aufwand bei der Herstellung dieser Darreichungsformen, stehen eindeutige Vorteile gegenüber. Bei direkter intravenöser Applikation eines Wirkstoffs lässt sich der Metabolismus im Gastrointestinaltrakt weitgehend umgehen, dem oral applizierte Wirkstoffe stets unterworfen sind. So wird insbesondere der sogenannte "First-Pass-Effekt" durch die Leberpassage minimiert. Manche Wirkstoffe lassen sich ausschliesslich nur intravenös applizieren, die oral ungenügend resorptionsfähig wären. Andere Wirkstoffe lassen sich intravenös in einer geringer wirksamen Dosis applizieren als für die orale Applikation erforderlich ist. Generell steht bei lebensbedrohenden Krankheiten, wie Tumorerkrankungen, die intravenöse Applikation im Vordergrund, da die Problematik der Resorption durch den Gastrointestinaltrakt verbunden mit unerwünschtem Metabolismus nicht in Kauf genommen werden kann.

Für die wichtige Wirkstoffgruppe der Staurosporine bzw. Derivate des Staurosporins hat bisher noch keine geeignete intravenöse Darreichungsform zur Verfügung gestanden. Der vorliegenden Erfindung liegt die Aufgabe zugrunde, für Staurosporinderivate, insbesondere für N-Benzoyl-staurosporin, eine geeignete intravenöse Darreichungsform zur Verfügung zu stellen.

Zahlreiche Publikationen schlagen verschiedene Möglichkeiten vor, einen schwerlöslichen Wirkstoff in eine für intravenöse Formulierungen geeignete, löslichere Form zu überführen. Dies kann beispielsweise mit Hilfe sogenannter Solubilisatoren, wie 1,2-Propylenglycol oder Polyethylenglycol 300-400, geschehen. Bei mangelnder Löslichkeit trotz Verwendung der wenigen in den nationalen Pharmakopöen und Arzneibüchem zugelassenen Solubilisatoren bieten sich gemäss Stand der Technik feindisperse Systeme auf der Basis von Lipidgemischen an. Darin ist der schwerlösliche Wirkstoff in Lipidpartikeln mit einer Teilchengrösse kleiner als 1 µm eingeschlossen und bildet mit der wässrigen Trägerflüssigkeit ein kolloiddisperses oder vorzugsweise feindisperses System, das zwar keine echte molekular disperse Lösung darstellt, aber für eine intravenöse Darreichungsform trotzdem ausreichend homogen ist. Zahlreiche Publikationen schlagen die Verkapselung von schwerlöslichen Wirkstoffen in Mizellen, Mischmizellen, Umkehrmizellen oder unilamellaren oder multilamellaren Liposomen vor.

In der Europäischen Patentschrift Nr. 406 162 (Weder et al.) ist ein Verfahren in einem Hochdruckhomogenisator zur Herstellung von Nanoemulsionen mit einer durchschnittlichen Teilchengrösse kleiner als 200 nm der dispergierten Lipide und die Verwendbarkeit dieser Nanoemulsionen für die Herstellung von intravenösen Darreichungsformen beschrieben.

Es wurde überraschenderweise gefunden, dass das besonders schwerlösliche Staurosporin bzw. seine Derivate sich in Nanoemulsionen in der für intravenöse Darreichungsformen erforderlichen Homogenität solubilisieren lassen.

Gegenstand der vorliegenden Erfindung ist eine Formulierungsgrundlage für die Herstellung einer pharmazeutischen Zusammensetzung für die intravenöse Applikation von Staurosporin-Derivaten enthaltend:
a) ein in Wasser schwerlösliches Staurosporin-Derivat;
b) mindestens ein im wesentlichen reines Phospholipid der Formel: 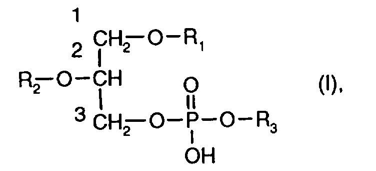 worin R₁ C₁₀₋₂₀-Acyl, R₂ Wasserstoff oder C₁₀₋₂₀-Acyl, R₃ Wasserstoff, 2-Trimethylamino-1-ethyl, 2-Amino-1-ethyl, C₁₋₄-Alkyl, C₁₋₅-Alkyl substituiert durch Carboxy, C₂₋₅-Alkyl substituiert durch Hydroxy, C₂₋₅-Alkyl substituiert durch Carboxy und Hydroxy oder C₂₋₅-Alkyl substituiert durch Carboxy und Amino, die Inositol- oder die Glycerylgruppe bedeuten, oder Salze dieser Verbindungen;
c) ein Triglycerid der Formel: 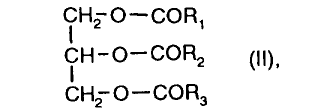 worin R₁, R₂ und R₃ C₈₋₂₄-Acyl bedeuten;
d) einen Partialfettsäureester des Polyoxyethylensorbitans;
e) Wasser als Trägerflüssigkeit in der für die intravenöse Applikation erforderlichen Reinheit; und gegebenenfalls
f) für Injektabilia geeignete wasserlösliche Hilfsstoffe.

Die weiter vom definierte pharmazeutische Zusammensetzung zeichnet sich durch günstige Phaseneigenschaften des solubilisierten Wirkstoffs aus. So ist bei vorhandener Opaleszenz und Transparenz im Gegenlicht nur an einer äusserst geringen milchigen Trübung zu erkennen, dass die Nanoemulsion noch physikalische Unterschiede gegenüber dem Idealzustand einer echten molekularen Lösung aufweist. Diese Unterschiede sind aber aufgrund der guten Homogenitätseigenschaften der Nanoemulsion hinnehmbar, die beispielsweise an einer überraschend hohen Lagerstabilität, z.B. keine Entmischung nach sechsmonatiger Lagerung bei 2°-8°C (durch Extrapolation zu erwartende Stabilität länger als zwei Jahre), und überraschend günstigen toxikologischen Eigenschaften nachweisbar sind. So waren 14-tägige toxikologische Untersuchungen an Ratten ohne auffälligen Befund.

Eine besonders bevorzugte Ausführungsform betrifft eine Formulierungsgrundlage für die Herstellung einer pharmazeutischen Zusammensetzung enthaltend:
a) den Wirkstoff N-Benzoyl-Staurosporin;
b) gereinigtes Lecithin aus Sojabohnen;
c) ein Triglycerid aus der Gruppe der Neutralöle;
d) Polyoxyethylen-(20)-sorbitanmonooleat;
e) Wasser als Trägerflüssigkeit in der für die intravenöse Applikation erforderlichen Reinheit; und gegebenenfalls
f) für Injektabilia geeignete wasserlösliche Hilfsstoffe.

Die weiter vom und im folgenden genannten Begriffe werden im Rahmen der Beschreibung der vorliegenden Erfindung wie folgt definiert:

Der Begriff Formulierungsgrundlage definiert ein Gemisch der genannten Komponenten b) bis f) ohne die Aktivsubstanz Staurosporinderivat - Komponente a) - . Die Formulierungsgrundlage ist als Vorstufe zur Herstellung der weiter vom beschriebenen intravenös applizierbaren pharmazeutischen Zusammensetzung geeignet.

Komponente a): Ein in Wasser schwerlösliches Staurosporinderivat ist beispielsweise in der U.S.Patentschrift 5 093 330 beschrieben und leitet sich durch zusätzliche Substitution des freien Wasserstoffatoms am Stickstoff des N-Methylamino-Substituenten ab. Für Staurosporinderivate ist die schlechte Löslichkeit in Wasser charakteristisch, was sie für intravenöse Darreichungsformen ungeeignet erscheinen lässt. So besitzt das besonders wirksame N-Benzoyl-staurosporin bei Raumtemperatur eine Wasserlöslichkeit von weniger als 0,1 mg/l.

Geeignete Staurosporin-Derivate sind beispielsweise N-(3-Nitrobenzoyl)-staurosporin, N-(3-Fluorbenzoyl)-staurosporin, N-Trifluoracetylstaurosporin, N-Phenylcarbamoylstaurosporin, N-(3-Carboxypropionyl)-staurosporin, N-Methylaminothiocarbonylstaurosporin, N-tert-Butoxycarbonylstaurosporin, N-(4-Carboxybenzoyl)-staurosporin, N-(3,5-Dinitrobenzoyl)-staurosporin, N-(2-Aminoacetyl)-staurosporin, N-Alanylstaurosporin sowie pharmazeutisch annehmbare Salze dieser Derivate. Besonders bevorzugt ist das N-Benzoylstaurosporin-Derivat.

Komponente b): Die Nomenklatur der Phospholipide (I) und die Bezifferung der C-Atome erfolgt anhand der in Eur. J. of Biochem. 79, 11-21 (1977) "Nomenclature of Lipids" von der IUPAC-IUB Commission on Biochemical Nomenclature (CBN) gegebenen Empfehlungen (sn-Nomenklatur, stereospecific numbering).

R₁ und R₂ mit den Bedeutungen C₁₀₋₂₀-Acyl sind vorzugsweise geradkettiges C₁₀₋₂₀-Alkanoyl mit einer geraden Anzahl an C-Atomen und geradkettiges C₁₀₋₂₀-Alkenoyl mit einer Doppelbindung und einer geraden Anzahl an C-Atomen.

Geradkettiges C₁₀₋₂₀-Alkanoyl R₁ und R₂ mit einer geraden Anzahl an C-Atomen sind beispielsweise n-Dodecanoyl, n-Tetradecanoyl, n-Hexadecanoyl oder n-Octadecanoyl.

Geradkettiges C₁₀₋₂₀-Alkenoyl R₁ und R₂ mit einer Doppelbindung und einer geraden Anzahl an C-Atomen sind beispielsweise 6-cis- oder 6-trans-, 9-cis- oder 9-trans-Dodecenoyl, -Tetradecenoyl, -Hexadecenoyl, -Octadecenoyl oder -lcosenoyl, insbesondere 9-cis-Octadecenoyl (Oleoyl), femer 9,12-cis-Octadecadienoyl oder 9,12,15-cis-Octadecatrienoyl.

Ein Phospholipid (I), worin R₃ 2-Trimethylamino-1-ethyl bedeutet, wird mit dem Trivialnamen Lecithin und ein Phospholipid (I), worin R₃ 2-Amino-1-ethyl bedeutet, mit dem Trivialnamen Kephalin bezeichnet. Geeignet sind beispielsweise natürlich vorkommendes Kephalin oder Lecithin, z.B. Kephalin oder Lecithin aus Sojabohnen oder Hühnerei mit verschiedenen oder identischen Acylgruppen R₁ und R₂, oder Mischungen davon.

Das Phospholipid (I) kann aber auch synthetischen Ursprungs sein. Unter dem Begriff synthetisches Phospholipid definiert man Phospholipide, welche bezüglich R₁ und R₂ eine einheitliche Zusammensetzung haben. Solche synthetischen Phospholipide sind vorzugsweise die weiter vom definierten Lecithine und Kephaline, deren Acylgruppen R₁ und R₂ eine definierte Struktur haben und von einer definierten Fettsäure mit einem Reinheitsgrad höher als ca. 95% abgeleitet sind. R₁ und R₂ können gleich oder verschieden und ungesättigt oder gesättigt sein. Bevorzugt ist R₁ gesättigt, z.B. n-Hexadecanoyl, und R₂ ungesättigt, z.B. 9-cis-Octadecenoyl (Oleoyl).

Der Begriff "natürlich vorkommendes" Phospholipid (I) definiert Phospholipide, welche bezüglich R₁ und R₂ keine einheitliche Zusammensetzung haben. Solche natürlichen Phospholipide sind ebenfalls Lecithine und Kephaline, deren Acylgruppen R₁ und R₂ strukturell undefinierbar und von natürlich vorkommenden Fettsäuregemischen abgeleitet sind.

Die Forderung "im wesentlichen reines" Phospholipid (I) definiert einen Reinheitsgrad von mehr als 90%(Gew.), vorzugsweise mehr als 95%, des Phospholipids (I), welcher anhand geeigneter Bestimmungsmethoden, z.B. papierchromatographisch, mit Dünnschichtchromatographie, mit HPLC oder enzymatischem Farbtest, nachweisbar ist.

In einem Phospholipid (I) ist R₃ mit der Bedeutung C₁₋₄-Alkyl beispielsweise Methyl oder Ethyl. Die Bedeutung Methyl ist bevorzugt.

R₃ mit den Bedeutungen C₁₋₅-Alkyl substituiert durch Carboxy, C₂₋₅-Alkyl substituiert durch Hydroxy oder C₂₋₅-Alkyl substituiert durch Carboxy oder Hydroxy sind beispielsweise 2-Hydroxyethyl, 2,3-Dihydroxy-n-propyl, Carboxymethyl, 1- oder 2-Carboxyethyl, Dicarboxymethyl, 2-Carboxy-2-hydroxyethyl oder 3-Carboxy-2,3-dihydroxy-n-propyl.

R₃ mit der Bedeutung C₂₋₅-Alkyl substituiert durch Carboxy und Amino ist z.B. 3-Amino-3-carboxy-n-propyl oder 2-Amino-2-carboxy-n-propyl, vorzugsweise 2-Amino-2-carboxyethyl. Phospholipide (I) mit diesen Gruppen können in Salzform, z.B. als Natrium- oder Kaliumsalz, vorliegen.

Phospholipide (I), worin R₃ die Inositol- oder die Glycerylgruppe bedeutet, sind unter den Bezeichnungen Phosphatidylinositol und Phosphatidylglycerol bekannt.

Für die Acylreste in den Phospholipiden (I) sowie in den Triglyceriden (II) sind auch die in Klammern angegebenen Bezeichnungen gebräuchlich:

9-cis-Dodecenoyl (Lauroleoyl), 9-cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl (Oleoyl), 9-trans-Octadecenoyl (Elaidoyl), 9,12-cis-Octadecadienoyl (Linoleoyl), 9,12,15-cis-Octadecatrienoyl (Linolenoyl),11-cis-Octadecenoyl (Vaccenoyl), 9-cis-lcosenoyl (Gadoleoyl), 5,8,11,14-cis-Eicosatetraenoyl (Arachidonoyl), n-Dodecanoyl (Lauroyl), n-Tetradecanoyl (Myristoyl), n-Hexadecanoyl (Palmitoyl), n-Octadecanoyl (Stearoyl), n-lcosanoyl (Arachidoyl), n-Docosanoyl (Behenoyl), n-Tetracosanoyl (Lignoceroyl).

Ein Salz des Phospholipids(l) ist vorzugsweise pharmazeutisch annehmbar. Salze definieren sich durch die Existenz von salzbildenden Gruppen im Substituenten R₃ sowie durch die freie Hydroxygruppe am Phosphor. Möglich ist ebenfalls die Bildung von inneren Salzen. Bevorzugt sind Alkalimetallsalze, insbesondere Natriumsalze.

In einer besonders bevorzugten Ausführungsform verwendet man gereinigtes Lecithin aus Sojabohnen.

Komponente c): In einem als Komponente c) verwendeten Triglycerid der Formel II bedeuten R₁, R₂ und R₃ geradkettiges C₈₋₂₄-Acyl mit gerader Anzahl an C-Atomen, insbesondere n-Octanoyl, n-Dodecanoyl, n-Tetradecanoyl, n-Hexadecanoyl, n-Octadecanoyl, 9-cis-Dodecenoyl, 9-cis-Tetradecenoyl, 9-cis-Hexadecenoyl, 9-cis-Octadecenoyl oder 9-cis-lcosenoyl. Die Bedeutungen von R₁, R₂ und R₃ können identisch oder verschieden sein, wobei die individuellen Gruppen R₁, R₂ und R₃ selbst strukturell einheitlich definiert sind. Dies ist für synthetische oder halbsynthetische Triglyceride charakteristisch. R₁, R₂ und R₃ können aber auch aus verschiedenen Acylgruppen unterschiedlicher Struktur bestehen. Dies ist für Triglyceride natürlichen Ursprungs charakteristisch.

Ein Triglycerid der FormellI ist ein halbsynthetisches oder synthetisches, im wesentlichen reines Triglycerid oder ein pharmazeutisch gebräuchliches Triglycerid natürlichen Ursprungs. Bevorzugt ist ein Triglycerid natürlichen Ursprungs, z.B. Erdnuss-, Sesam-, Sonnenblumen-, Oliven-, Maiskeim-, Soja-, Rizinus-, Baumwollsamen-, Raps-, Distel-, Traubenkern-, Fisch- oder Kokosöl. In einer besonders bevorzugten Ausführungsform der Erfindung verwendet man ein mit dem Begriff Neutralöl definiertes Triglycerid mit verschiedenen Acylgruppen unterschiedlicher Struktur, z.B. ein Triglycerid der fraktionierten Kokosfettsäuren C₈-C₁₀ vom Typ Miglyol®, z.B. MIGLYOL 812.

Komponente d): Der genannte Partialfettsäureester des Polyoxyethylensorbitans besteht vorzugsweise aus einem im wesentlichen reinen oder dem Gemisch verschiedener Ester des Sorbitans, worin die Struktur der Fettsäuregruppen und die Länge der Polyoxyethylenketten variieren. Das Sorbitan ist vorzugsweise durch drei Polyoxyethylenketten veräthert und durch eine Fettsäuregruppe verestert. Das Sorbitan kann aber auch nur durch ein oder zwei Polyoxyethylenketten veräthert und entsprechend durch zwei oder drei Fettsäuregruppen verestert sein. Insgesamt ist der Sorbitangrundkörper durch mindestens zwei und maximal vier hydrophile Gruppen substitutiert, wobei unter dem Begriff hydrophile Gruppe die Polyoxyethylenketten und Fettsäuregruppen zusammengefasst werden.

Die Polyoxyethylenkette ist geradkettig und weist vorzugsweise 4-10, insbesondere 4-8, Ethylenoxideinheiten auf. Die Estergruppen am Sorbitangrundkörper sind von einer gesättigten oder ungesättigten, geradkettigen Carbonsäure und gerader Anzahl von 8-20 C-Atomen abgeleitet. Die von dieser Carbonsäure abgleitete Estergruppe ist vorzugsweise geradkettig mit 12, 14, 16 und 18 C-Atomen, z.B. n-Dodecanoyl, n-Tetradecanoyl, n-Hexadecanoyl oder n-Octadecanoyl. Die von einer ungesättigten Carbonsäure mit gerader Anzahl von 8-20 C-Atomen abgeleitete Estergruppe ist vorzugsweise geradkettig mit 12, 14, 16 und 18 C-Atomen, z.B. Oleoyl. Die genannten Ester des Sorbitans erfüllen die in der Britischen Pharmakopöe (spezielle Monographie) oder Ph.Helv.VI genannten Angaben. Es gelten insbesondere die von den genannten Herstellem publizierten Produktbeschreibungen mit den Angaben auf Datenblättem für das betreffende Produkt, insbesondere Spezifikationen wie Form, Farbe, HLB-Wert, Viskosität, Steigschmelzpunkt und Löslichkeit.

Geeignete Partialfettsäureester des Polyoxyethylensorbitans sind unter dem Wortzeichen Tween® der Fa.ICI kommerziell erhältlich und den chemischen Bezeichnungen Polyoxyethylen-(20 oder 4)-sorbitanmonolaurat (TWEEN 20 und 21), Polyoxyethylen-(20)-sorbitanmonopalmitat oder -monostearat (TWEEN 40 und 60), Polyoxyethylen-(4 oder 20)-sorbitanmonostearat oder -tristearat (TWEEN 61 und 65), Polyoxyethylen-(20 oder 5)-sorbitan-monooleat (TWEEN 80 oder 81) oder Polyoxyethylen-(20)-sorbitantrioleat (TWEEN 85) bekannt.

In einer besonders bevorzugten Ausführungsform der Erfindung verwendet man als Komponente d) Polyoxyethylen-(20)-sorbitanmonooleat (TWEEN 80).

Die Komponente e), Wasser als Trägerflüssigkeit in der für die intravenöse Applikation erforderlichen Reinheit, ist nach den Vorschriften der nationalen Pharmakopöen keim- und pyrogenfrei.

Die Komponente f), für Injektabilia geeignete wasserlösliche Hilfsstoffe, ist in der pharmazeutischen Zusammensetzung wahlweise vorhanden. Geeignet sind Hilfsstoffe zur Herstellung von isotonischen Bedingungen, z.B. ionische Hilfsstoffe, z.B. Natriumchlorid, oder andere wasserlösliche Hilfsstoffe, z.B. Sorbitan, Mannit, Glucose, Lactose oder Fructose.

Ebenfalls Gegenstand der vorliegenden Erfindung ist die Weiterverarbeitung der Formulierungsgrundlage zur Herstellung einer pharmazeutischen Zusammensetzung, welche dadurch gekennzeichnet ist, dass man eine Liposomendispersion enthaltend das Phospholipid der Formel I und gegebenenfalls wasserlösliche Hilfsstoffe herstellt, eine ölige, homogene Mischung bestehend aus dem schwerlöslichen Staurosporinderivat, dem Triglycerid der Formel II und dem Partialfettsäureester des Polyoxyethylensorbitans herstellt, die wässrige Liposomendispersion und die ölige, homogene Mischung miteinander vermischt, die erhältliche Mischung den Bedingungen der Hochdruckhomogenisation unterwirft und die erhältliche klare Dispersion folgenden Nachoperationen unterzieht:
α) Zusatz einer weiteren Menge Wasser als Trägerflüssigkeit sowie gegebenenfalls weiterer wasserlöslicher Hilfsstoffe, welche für Injektabilia geeignet sind, Filtration und gegebenenfalls Dialyse der klaren Dispersion; oder
β) Filtration und gegebenenfalls Dialyse und anschliessende Überführung der erhältlichen klaren Dispersion in ein Trockenpräparat, gegebenenfalls unter Zusatz von wasserlöslichen Hilfsstoffen, und Rekonstitution des Trockenpräparats zu einer injizierbaren Dispersion.

In einer besonders bevorzugten Verfahrensvariante stellt man eine intravenös applizierbare Nanoemulsion mit dem in Wasser schwerlöslichen Staurosporinderivat N-Benzoylstaurosporin her.

Gegenstand der vorliegenden Erfindung ist die für dieses Verfahren verwendbare Formulierungsgrundlage enthaltend:
b) mindestens ein im wesentlichen reines Phospholipid der Formel: 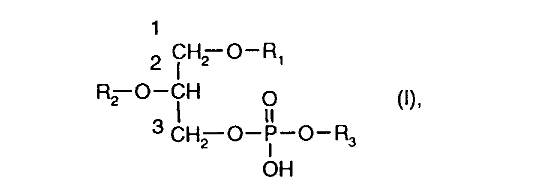 worin R₁, R₂ und R₃ die genannten Bedeutungen haben, oder Salze dieser Verbindungen;
c) ein Triglycerid der Formel: 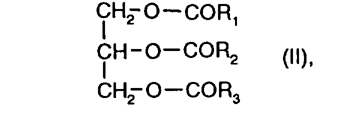 worin R₁, R₂ und R₃ die genannten Bedeutungen haben;
d) einen Partialfettsäureester des Polyoxyethylensorbitans;
e) Wasser als Trägerflüssigkeit in der für die intravenöse Applikation erforderlichen Reinheit; und gegebenenfalls
f) für Injektabilia geeignete wasserlösliche Hilfsstoffe;

Diese Formulierungsgrundlage ist sowohl für intravenöse als auch für solche Darreichungsformen geeignet, worin die Solubilisierung eines schwerlöslichen Wirkstoffs erforderlich ist, z.B. für Kapselfüllungen, Tropfen, Lotionen oder Emulsionen für Salben, Cremes etc..

Letzteren kann man auch noch die für diese Darreichungsformen charakteristischen weiteren Hilfsstoffe zufügen. Die Formulierungsgrundlage ist sowohl für die Solubilisierung von schwerlösllichen Staurosporin-Derivaten im Sinne der beschriebenen Aufgabenstellung als auch für die Solubilisierung von anderen schwerlöslichen Wirkstoffen verwendbar. Die Herstellung der Formulierungsgrundlage erfolgt in analoger Weise zur Herstellung der pharmazeutischen Zusammensetzung. Soll die Formulierungsgrundlage isoliert werden, wird kein Wirkstoff hinzugesetzt.

Besonders bevorzugt ist die Formulierungsgrundlage enthaltend:
b) gereinigtes Lecithin aus Sojabohnen;
c) ein Triglycerid aus der Gruppe der Neutralöle;
d) Polyoxyethylen-(20)-sorbitanmonooleat;
e) Wasser als Trägerflüssigkeit in der für die intravenöse Applikation erforderlichen Reinheit; und gegebenenfalls
f) für Injektabilia geeignete wasserlösliche Hilfsstoffe.

Die Herstellung der wässrigen Liposomendispersion enthaltend die Phospholipidkomponente b) der Formel I erfolgt unter Anwendung eines der an sich bekannten Herstellungsverfahren von Liposomen, z.B. indem man eine wässrige, grobe Dispersion enthaltend die Phospholipidkomponente b) durch intensives Schütteln mit einem Dispergator, z.B. mit einem Vortex-Mischer, statischen Mischem oder Dispergatoren vom Typ POLYTRON (Kinematica AG, Littau CH) oder Dispergatoren der Fa. EKA (DE-Staufen) homogenisiert. Dabei erfolgt die Bildung von Liposomen, welche gross, klein, unilamellar oder multilamellar sein können. Es können ca. 0,1 bis 50 Gewichtsprozent, bezogen auf das Gesamtgewicht der wässrigen Dispersion in dieser Vorstufe des Verfahrens, vorzugsweise ca. 2 bis 20 Gewichtsprozent, in wässriger Phase dispergiert werden. Bei der Herstellung der Liposomendispersion ist die sogenannte Phasenübergangstemperatur (gelförmig/flüssigkristallin) der verwendeten Phospholipide kritisch. Man dispergiert vorzugsweise bei Temperaturen, worin die Phospholipide in flüssigkristallinem Zustand vorliegen, also oberhalb der sogenannten Phasenübergangstemperatur. Besonders geeignet sind Phospholipide, die bei Raumtemperatur oder niedrigeren Temperaturen in flüssigkristallinem Zustand vorliegen. Gegebenenfalls stellt man die Liposomen unter Kühlen und/oder Inertgasatmosphäre her.

Die Grösse und Struktur (multilamellar - unilamellar) der gebildeten Liposomen in dieser Vorstufe ist u.a. von der eingesetzten Menge der Phospholipidkomponente und der Wahl des betreffenden Verfahrens abhängig. Bei Schütteln oder Rühren, z.B. mit konventionellen Rührem mit Propeller oder Flügelblatt oder mit einem Magnetrührer, erhält man Dispersionen mit einem hohen Anteil an grossen multilamellaren Liposomen. Die Erhöhung der Rührfrequenz oder der Übergang zu Phasenmischem mit hohen Scherkräften bewirkt eine Erhöhung des Anteils an kleinen, multilamellaren Liposomen. Die Behandlung mit Ultraschall ergibt einen hohen Anteil an unilamellaren Lipsomen in der Dispersion.

Die Herstellung der öligen, homogenen Mischung bestehend aus dem Wirkstoff Staurosporin-Derivat, insbesondere N-Benzoyl-staurosporin, dem Triglycerid der Formel II und dem Partialfettsäureester des Polyoxyethylensorbitans erfolgt durch Vermischen der Komponenten und Schütteln oder Rühren, z.B. mit konventionellen Rührem mit Propeller oder Flügelblatt oder mit einem Magnetrührer oder Phasenmischem, die z.B. von der Firma Vortex im Handel erhältlich sind. Diese Verfahrensvariante führt zur Herstellung der wirkstoffhaltigen pharmazeutischen Zusammensetzung. Wird nur die Formulierungsgrundlage isoliert, setzt man keinen Wirkstoff hinzu. Um eine besonders homogene Mischung zu erhalten, rührt man hochtourig, z.B. mit Rührern der Fa. Polytron, z.B. POLYTRON PT 3000 oder DH 30/30.

Die Weiterverarbeitung zur pharmazeutischen Zusammensetzung erfolgt durch Vermischen der wässrigen Liposomendispersion mit der öligen, homogenen Mischung (Olphase).

Vorzugsweise vermischt man 0,05 bis 0,4 Gewichtsteile Phospholipid (I) bezogen auf 1 Gewichtsteil der Ölphase. Beim Vermischen bildet sich zunächst eine Emulsion, welche man in einem Hochdruckhomogenisator weiterbehandelt. Unter den Bedingungen der Hochdruckhomogenisation bildet sich eine Dispersion mit physikochemischen Eigenschaften, die mit dem Begriff Nanoemulsion definierbar ist. Eine Nanoemulsion lässt sich als kolloid-hochdisperses Zweiphasen-System auffassen. Aufgrund von Laser-Streulichtmessungen und Aufnahmen im Elektronenmikroskop sind die in der Dispersion vorhandenen amphiphilen Partikel von anderen Gebilden wie Flüssigkristallen, Mizellen, Umkehrmizellen oder Liposomen unterscheidbar. Für die statistische Mehrzahl von mehr als 90%, vorzugsweise mehr als 95%, ist eine mittlere Partikelgrösse kleiner als 20 nm charakteristisch.

Als Hochdruckhomogenisatoren eignen sich die im Handel üblichen Fabrikate, z.B. der Firmen Rannie (APV Rannie AS, Albertslund DK) oder Gaulin (APV Gaulin Intern. BV, Hilversum NL), insbesondere das Modell Rannie High Pressure Laboratory Homogeniser Mini-Lab, Typ 8.30 H. Als Druck wählt man einen Bereich von ca. 500-1000 bar, vorzugsweise ca. 600-800 bar. Als Verfahrenstemperatur ist ebenfalls der flüssigkristalline Bereich der in der Nanoemulsion vorhandenen Phospholipide von ca. 0° bis 40°C, insbesondere 20° bis 30°C, bevorzugt.

Zur Charakterisierung der erhältlichen Nanoemulsionen sind an sich bekannte Methoden geeignet, z.B. optische Beurteilung: schwache bis starke Opaleszenz des Präparats ist leicht erkennbar (Hinweis auf durchschnittliche Partikelgrösse kleiner als 50 nm); Laser-Lichtstreuung (Bestimmung der Partikelgrösse und Homogenität); Elektronenmiskroskopie (Gefrierbruch- und Negativkontrasttechnik).

### Nachoperationen:

Der Nanoemulsion kann man die gewünschte Menge Wasser, welches in einer für Injektionen geforderten Reinheit vorliegen muss, hinzusetzen, so dass die Nanoemulsion nach Wahl einer für solche Dispersionen geeigneten Filtrationsmethode, z.B. steriler Gelfiltration, z.B. mit Sepharose® oder Sephacryl® (Pharmacia) als Träger, oder vorzugsweise Sterilfiltration (0,2 µm), z.B. mit PAL-Filter (Gelman) und gegebenenfalls nach Zusatz von weiteren wasserlöslichen Hilfsstoffen, die für intravenöse Darreichungsformen verwendbar sind, direkt applizierbar ist. Besonders durch Sterilfiltration kann man alle in der Dispersion befindlichen grösseren Partikel mit einem Durchmesser grösser als ca. 200 nm, sowie Schweb- und Feststoffe, überschüssige, dispergierte Lipide, welche in hochmolekularen Aggregaten vorliegen können, abtrennen und so eine Nanoemulsion mit einer Fraktion von hydrophilen Partikeln mit relativ einheitlicher Grösse herstellen. Altemativ oder zusätzlich zur Sterilfiltration kann man die Nanoemulsion zwecks Reinigung dialysieren und/oder ultrafiltrieren.

Die weiter vom beschriebenen pharmazeutischen Zusammensetzungen, welche durch Weiterverarbeitung der Formulierungsgrundlage erhältlich sind, sind als intravenös verabreichbare Arzneimittel zur Behandlung von Krankheiten, die von malignem Zellwachstum verursacht werden, verwendbar. Insbesondere eignen sie sich als Tumorhemmer, als Antiinflammatorika, als Antibiotika, bei der Behandlung von Arteriosklerose, oder sind bei diversen Erkrankungen des kardiovaskulären Systems und des zentralen Nervensystems therapeutisch anwendbar.

Das folgende Beispiel illustriert die Erfindung:

### Beispiel:

a) Rezeptur für eine Formulierung (Dosiseinheit):

| | |
|---|---|
| 1,0 mg | N-Benzoyl-staurosporin |
| 5,5 mg | Lecithin aus Sojabohnenöl (LIPOID S 100) |
| 11,1 mg | Neutralöl MIGLYOL 812 |
| 16,6 mg | PS 80: TWEEN 80. |

b) Ansatzmenge für 5 kg-Ansatz:

| | |
|---|---|
| 9,0 g | N-Benzoyl-staurosporin |
| 50 0 g | Lecithin aus Sojabohnenöl (Lipoid S 100) |
| 100,0 g | Neutralöl MIGLYOL 812 |
| 150,0 g | PS 80: TWEEN 80. |

Man stellt unter Verwendung eines Rührers der Fa. Polytron, z.B. POLYTRON PT 3000 oder DH 30/30, eine ölige, homogene Mischung bestehend aus N-Benzoyl-staurosporin, dem Neutralöl und TWEEN 80 her.

In einem Rundkolben wird das Lecithin aus Sojabohnenöl (Lipoid S 100) vorgelegt und mit Aqua inject. vermischt. Man dispergiert mit einem Dispergator vom Typ POLYTRON (pH 6-8, Raumtemperatur, 10000 UpM, Endtemperatur 25-27°C, 7 Min..

Nach Vermischen der öligen Suspension mit der wässrigen Liposomendispersion unterwirft man das Gemisch in einem Rannie High Pressure Laboratory Homogeniser Mini-Lab, Typ 8.30 H, der Hochdruckhomogenisation jeweils 25 Minuten lang in drei Zyklen. Als Druck stellt man ca.600 bar ein. Als Verfahrenstemperatur wählt man den Bereich von 30-33°C. Die erhaltene Nanoemulsion wird anschliessend über 0,2 µm PAL-Filter sterilfiltriert.

## Patentansprüche

1. Formulierungsgrundlage enthaltend:
b) mindestens ein im wesentlichen reines Phospholipid der Formel: 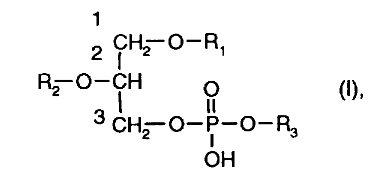 worin R₁ C₁₀₋₂₀-Acyl, R₂ Wasserstoff oder C₁₀₋₂₀-Acyl, R₃ Wasserstoff, 2-Trimethylamino-1-ethyl, 2-Amino-1-ethyl, C₁₋₄Alkyl, C₁₋₅-Alkyl substituiert durch Carboxy, C₂₋₅-Alkyl substituiert durch Hydroxy, C₂₋₅-Alkyl substituiert durch Carboxy und Hydroxy oder C₂₋₅-Alkyl substituiert durch Carboxy und Amino, die Inositol- oder die Glycerylgruppe bedeuten, oder Salze dieser Verbindungen;
c) ein Triglycerid der Formel: 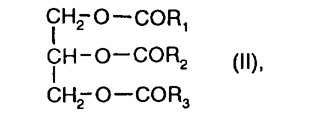 worin R₁, R₂ und R₃ C₈₋₂₄-Acyl bedeuten;
d) einen Partialfettsäureester des Polyoxyethylensorbitans;
e) Wasser als Trägerflüssigkeit in der für die intravenöse Applikation erforderlichen Reinheit; und gegebenenfalls
f) für Injektabilia geeignete wasserlösliche Hilfsstoffe.

2. Formulierungsgrundlage gemäss Anspruch 1 enthaltend:
b) gereinigtes Lecithin aus Sojabohnen;
c) ein Triglycerid aus der Gruppe der Neutralöle;
d) Polyoxyethylen-(20)-sorbitanmonooleat;
e) Wasser als Trägerflüssigkeit in der für die intravenöse Applikation erforderlichen Reinheit; und gegebenenfalls
f) für Injektabilia geeignete wasserlösliche Hilfsstoffe.

3. Verfahren zur Herstellung einer pharmazeutischen Formulierungsgrundlage gemäss Anspruch 1 für ein intravenös applizierbares,schwerlöslichen Staurosporin-Derivat, dadurch gekennzeichnet, dass man eine Liposomendispersion enthaltend das Phospholipid der Formel I und gegebenenfalls wasserlösliche Hilfsstoffe herstellt, eine ölige, homogene Mischung bestehend aus dem Triglycerid der Formel II und dem Partialfettsäureester des Polyoxyethylensorbitans herstellt, die wässrige Liposomendispersion und die ölige, homogene Mischung miteinander vermischt, die erhältliche Mischung den Bedingungen der Hochdruckhomogenisation unterwirft, und die erhältliche klare Dispersion folgenden Nachoperationen unterzieht:
α) Zusatz einer weiteren Menge Wasser als Trägerflüssigkeit sowie gegebenenfalls weiterer wasserlöslicher Hilfsstoffe, welche für Injektabilia geeignet sind, Filtration und gegebenenfalls Dialyse der klaren Dispersion; oder
β) Filtration und gegebenenfalls Dialyse und anschliessende Überführung der erhältlichen klaren Dispersion in ein Trockenpräparat, gegebenenfalls unter Zusatz von wasserlöslichen Hilfsstoffen, und Rekonstitution des Trockenpräparats zu einer injizierbaren Dispersion.

4. Verfahren gemäss Anspruch 3, dadurch gekennzeichnet, dass man eine Nanoemulsion mit der Formulierungsgrundlage herstellt.

5. Das nach dem Verfahren gemäss Anspruch 3 erhältliche Konzentrat oder Trockenpräparat.
